# EUROPEAN PATENT SPECIFICATION

(11) **EP 2 415 400 B1**
(45) Date of publication and mention of the grant of the patent: **07.10.2015**
(21) Application number: 11176743.0
(22) Date of filing: 05.08.2011
(51) Int. Cl.: A61B 5/145

(54) **Mount unit, sensor unit, measurement apparatus, and sensor fixation method**
Montageeinheit, Sensoreinheit, Messvorrichtung und Sensorbefestigungsverfahren
Unité de montage, unité de détection, appareil de mesure et procédé de fixation de capteur

(30) Priority: 05.08.2010 JP 2010176465
(43) Date of publication of application: 08.02.2012
(73) Proprietor: ARKRAY, Inc., Minami-ku Kyoto-shi Kyoto 601-8045 (JP)
(72) Inventor: Shoshihara, Tomohiro, kyoto, Kyoto 602-0008 (JP); Yamamoto, Akihiro, Kyoto, Kyoto 602-0008 (JP)
(74) Representative: MacDougall, Alan John Shaw

(56) References cited:
- EP-A1- 1 707 114
- WO-A2-2009/154675
- US-B1- 7 460 897

## Description

### CROSS-REFERENCE TO RELATED APPLICATIONS

This application is based upon and claims the benefit of priority from Japanese patent application No. 2010-176465, filed on August 8, 2010.

### BACKGROUND OF THE INVENTION

### 1. Field of the Invention

The present invention relates to a mount unit used for fixing a sensor that can be subcutaneously embedded into a living body, a sensor unit that uses the mount unit, a measurement apparatus including the mount unit and the sensor unit, and a method for fixing the sensor.

### 2. Description of Related Art

For conventional blood sugar level measurements, it is necessary to prick the body of a patient with an instrument called a lancet and collect blood, each time measurement is performed. This is problematic in that a heavy physical burden is imposed on the patient and the measurement cannot be performed continuously. To solve such problems, a method called continuous glucose monitoring (CGM) has recently been proposed in which glucose concentrations in subcutaneous tissue are continuously measured.

In the CGM, a sensor is disposed such that a portion thereof is embedded under the skin of a patient, and a signal such as a current value depending on the glucose concentration in the interstitial fluid under the skin is continuously output by the sensor. Then, the signal is converted into a blood sugar level using a measuring device or the like. With the CGM, it is possible to continuously measure blood sugar levels (for example, see JP 2008-062072A (see FIGS. 14, 17, 26 to 28D, and 33). Although interstitial fluid is different from blood, it is considered that a glucose concentration in interstitial fluid reflects a glucose concentration in blood (blood sugar level). Therefore, the blood sugar level can be known by measuring a glucose concentration in interstitial fluid under the skin.

Since the CGW requires a portion of the sensor to be embedded subcutaneously into the patient, JP 2008-062072A discloses a device (embedded device) that can punch a sensor toward the skin together with a puncture needle and embed the sensor under the skin. The embedded device includes a mechanism that punches out the sensor together with the puncture needle using a spring or the like and then pulls back only the puncture needle. Here, the procedure for the sensor insertion disclosed in JP 2008-062072A will be described.

First, a mount unit for mounting the sensor is disposed on the skin of the patient. At this time, the mount unit is fixed to the patient's skin with a double-sided tape. Then, an embedded device on which the sensor and the puncture needle have been set is disposed in a predetermined position of the mount unit, and the sensor and the puncture needle together are punched into the skin by the embedded device. Thereafter, the puncture needle returns to its original position, but the sensor is positioned such that a portion provided with a connection terminal projects from the skin and the remaining portion is held under the skin.

After removal of the embedded device from the mount unit, a control unit for controlling the sensor is disposed on the mount unit. At this time, the portion of the sensor that is provided with the terminal (terminal portion) is sandwiched between the mount unit and the control unit, and the sensor is fixed to the patient with the mount unit. At the same time, the terminal of the control unit is connected to the sensor terminal projecting from the skin, completing a CGM system.

By subsequently operating the CGM system to perform sensing by the sensor, a signal obtained by the sensor is converted into a digital signal by the control unit, and the signal is further sent to an external measuring device by wireless or wired transmission. The measuring device calculates a specific glucose concentration based on the signal and displays the calculated value on a display screen.

Also, as described above, the sensor is fixed to the patient with the mount unit and the portion of the sensor that is embedded under the skin is prevented from moving accidentally. This is because if the portion of the sensor that is embedded under the skin moves and the wound formed in the skin by the embedded device is thus enlarged, the body of the patient functions so as to cover the sensor with protein in order to heal the wound, resulting in a state in which accurate measurement cannot be conducted.

Furthermore, since a sensor that has been covered with protein in this manner is unusable, removal of the sensor and insertion of a new sensor are necessary. However, this imposes considerable physical and financial burdens on the patient. Also, since whether a signal is output from the sensor can be known only after operation of the control unit and the measuring device, the patient may have to visit the medical institution again. Therefore, for the CGM system, the sensor that has been once embedded needs to be prevented from moving as much as possible.

In recent years, there is a demand for a sensor that can be embedded for a longer period of time. Accordingly, before the life of the sensor ends (before the sensor needs to be replaced), the double-sided tape used for fixing the mount unit to the patient may require replacement.

If double-sided tape that requires replacement in not replaced, hygiene problems such as a skin irritation and development of bacteria occur. Also, if the double-sided tape that requires replacement is not replaced, a reduction in adhesion of the double-sided tape, peeling of the double-sided tape or the like due to the turnover of the skin make the fixation of the sensor with the mount unit unstable. Consequently, the portion of the sensor that is embedded under the skin moves and the wound in the skin is thus enlarged, also causing the above-described problem of impairing accurate measurement. To solve these problems, the double-sided tape needs to be replaced regularly

However, the structure of the conventional mount unit used in the CGM system disclosed in JP 2008-062072A renders it difficult to replace the double-sided tape, with the sensor being embedded under the skin. Accordingly each time the double-sided tape is replaced, it is necessary to remove the sensor from the patient and replaced it with a new sensor, resulting in the need to perform pricking again. Also, despite applying the sensor that can be embedded for a long period of time, the sensor needs to be replaced each time the double-sided tape is replaced, and therefore sensors will be wastefully consumed.

US7460897 discloses a patient interface and a method of installing a patient interface for a spectroscopy kit on a patient. The patient interface includes a butterfly-shaped patient interface with a cap on top of the patient interface and in proximity to an optical head.

WO2009154675 discloses an introducer for introducing a sensor into the body of a patient. The introducer connects to a sensor hub. When the sensor hub and introducer are connected, the introducer needle is exposed. When the sensor hub and introducer are disconnected, a needle cover and the needle move with respect to each other so that the needle cover substantially covers the needle, protecting a user from being injured by the needle.

EP1707114 discloses a fluorescence measurement analytical kit that includes an adhesive fluorescence measurement patch, a fluorescent-light-emitting bead and a remote module. The adhesive fluorescence measurement patch has an adhesive sheet configured for removable adhesion to the user's body, a light emitter attached to the adhesive sheet, and a light detector attached to the adhesive sheet. The light emitter is configured for emitting light that is absorbed by the fluorescent light emitting bead while the light detector is configured for detecting fluorescent light emitted by the fluorescent light-emitting bead.

### SUMMARY OF THE INVENTION

An example of the object of the present invention is to provide a mount unit, a sensor unit, a measurement apparatus, and a sensor fixation method that can solve the above-described problems and allows for easy replacement of a fixing adhesive material layer, while preventing external force from being applied to the sensor.

To achieve the above-described object, a mount unit according to the present invention includes: a main body portion that holds a placement of a portion of a sensor under the skin of a living body; and two or more holding portions attached to the main body portion, wherein each of the two or more holding portions includes an adhesive material layer capable of adhering to the living body and is movable so as to allow for selection between a state in which the adhesive material layer is in contact with the living body and a state in which the adhesive material layer is separate from the living body, wherein the main body portion comprises a mechanism that can change a contact position of at least one of the holding portions that is in contact with the living body, in a state in which the position of the sensor is held. Accordingly, it is possible to suppress a skin irritation of the living body that can be caused by a long period of contact between the skin and the adhesive material layer.

According to the above-described feature, in the case where it is necessary to replace an adhesive material layer the adhesive material layer of one holding portion is kept adhering to the living body, and another holding portion is moved in this state to detach the adhesive material layer of the other holding portion from the living body, and the detached adhesive material layer can be replaced with a new adhesive material layer. That is, according to the present invention, it is possible to replace an old adhesive material layer, with the sensor being fixed. The present invention allows for easy replacement of the fixing adhesive material layer, while preventing external force being applied to the sensor.

In a preferred mode of the above-described mount unit according to the present invention, each of the two or more holding portions is attached to the main body portion via a hinge mechanism, and mobilization of the holding portions with the hinge mechanism allows for selection between a state in which the adhesive material layer is in contact with the living body and a state in which the adhesive material layer is separate from the living body. According to this preferred mode, it is possible to simplify the structure of the mount unit, thus realizing a reduction in manufacturing costs.

In another preferred mode of the above-described mount unit of the present invention, each of the two or more holding portions is attached to the main body portion so as to be movable in a direction toward the living body and a direction away from the living body, and movement of the holding portions allows for selection between a state in which the adhesive material layer is in contact with the living body and a state in which the adhesive material layer is separate from the living body. According to this mode as well, it is possible to simplify the structure of the mount unit, thus realizing a reduction in manufacturing costs.

In another preferred mode of the above-described mount unit of the present invention, each of the two or more holding portions is attached to the main body portion so as to further be rotatable with respect to the main body portion. According to this mode as well, it is possible to simplify the structure of the mount unit, thus realizing a reduction in manufacturing costs. Further, combination of this mode with the above-described modes can further simplify the replacement work of the adhesive material layer in a more reliable manner.

In addition, in the above-described mode, it is preferable that a plurality of the adhesive material layers are provided on an outer face of each of the two or more holding portions in the direction of rotation. In this case, the adhesive material layer can be replaced by simply rotating the holding portions.

In another preferred mode, a sensor unit includes: a sensor of which a portion can be placed under the skin of a living body; and the above described mount unit for fixing the sensor to the living body.

In another preferred mode, a measurement apparatus (for measuring numeric information relating to a substance contained in at least one of interstitial fluid and blood that are under the skin of a living body) includes: a sensor of which a portion can be placed under the skin and that generates a signal dependent on the numeric information; the above described mount unit for fixing the sensor to the living body; and a control unit that receives the signal generated by the sensor and executes processing that includes digital signal processing on the signal.

In a mode of the above-described measurement apparatus of the present invention, the control unit executes transmission processing for transmitting the signal that has been subjected to digital signal processing to an external measuring device. According to this mode, the signal can be converted into the numeric information by the external measuring device, and therefore the size of the control unit can be reduced.

Furthermore, in order to attain the above-described object, a sensor fixation method according to the embodiment is a method for fixing, to a living body, a sensor of which a portion can be placed under the skin of the living body, the method including the steps of:
(a) using a mount unit that includes a main body portion and two or more holding portions attached to the main body portion, each of the two or more holding portions including an adhesive material layer capable of adhering to the living body and attached to the main body portion so as to allow for selection between a state in which the adhesive material layers are in contact with the living body and a state in which the adhesive material layers are separate from the living body, and holding the sensor by the main body portion of the mount unit;
(b) fixing the mount unit to the living body by bringing the adhesive material layer of at least one of the two or more holding portions of the mount unit into contact with the living body; and,
(c) in a case where any of the adhesive material layers of the two or more holding portions needs to be replaced, moving the holding portion including the adhesive material layer that needs to be replaced, in a state in which the adhesive material layer of at least one of the holding portions is in contact with the living body, and detaching the adhesive material layer that needs to be replaced from the living body.

In a preferred mode of the above described sensor fixation method according to the present invention, each of the two or more holding portions is attached to the main body portion via a hinge mechanism, mobilization of the holding portions with the hinge mechanism allows for selection between a state in which the adhesive material layer is in contact with the living body and a state in which the adhesive material layer is separate from the living body, and, in step (c), the adhesive material layer that needs to be replaced is detached from the living body by mobilization of the holding portions with the hinge mechanism.

In another preferred mode of the above-described sensor fixation method of the present invention, each of the two or more holding portions is attached to the main body portion so as to be movable in a direction toward the living body and a direction away from the living body, movement of the holding portions allows for selection between a state in which the adhesive material layer is in contact with the living body and a state in which the adhesive material layer is separate from the living body, and, in step (c), the adhesive material layer that needs to be replaced is detached from the living body by movement of the holding portions.

In the above mode, it is more preferable that each of the two or more holding portions is attached to the main body portion so as to be rotatable with respect to the main body portion. In this case, it is further preferable that a plurality of the adhesive material layers are provided on an outer face of each of the two or more holding portions in the direction of rotation, and, in step (c), a new adhesive material layer replacing the adhesive material layer that needs to be replaced is faced toward the living body by rotation of the holding portions, and thereafter the new adhesive material layer is adhered to the living body by movement of the holding portions.

As described above, the mount unit, the sensor unit, the measurement apparatus, and the sensor fixation method according to the present invention allow for easy replacement of the fixing adhesive material layer, while preventing external force from being applied to the sensor.

### BRIEF DESCRIPTION OF THE DRAWINGS

FIGS. 1A to 1C show the configuration of a mount unit and a sensor unit according to Embodiment 1 of the present invention: FIG. 1A being a perspective view; FIG. 1B being a plan view; and FIG. 1C being a bottom view.
FIG. 2 is a perspective view showing a tip portion of a sensor used in Embodiment 1 of the present invention.
FIG. 3 is a perspective view showing a measurement apparatus according to Embodiment 1 of the present invention.
FIGS. 4A to 4D show a series of main steps constituting a sensor fixation method according to Embodiment 1 of the present invention.
FIGS. 5A to 5D show the configuration of a mount unit and a sensor unit according to Embodiment 2 of the present invention: FIG. 5A being a perspective view; FIG. 5B being a front view; FIG. 5C being a side view; and FIG. 5D being a bottom view
FIGS. 6A to 6D show a series of main steps constituting a sensor fixation method according to Embodiment 2 of the present invention.
FIG. 7 is a perspective view showing a modification of the mount unit according to Embodiment 2 of the present invention.
FIG. 8 shows the configuration of a mount unit according to Embodiment 3 of the present invention.
FIGS. 9A and 9B show a series of main steps constituting a sensor fixation method according to Embodiment 3 of the present invention.
FIGS. 10C and 10D show a series of main steps constituting a sensor fixation method according to Embodiment 3 of the present invention, which are executed after execution of the step shown in FIG. 9B.
FIG. 11 is a perspective view showing a mount unit according to Embodiment 4 of the present invention.
FIGS. 12A to 12C show a series of main steps constituting a sensor fixation method according to Embodiment 4 of the present invention.

### DETAILED DESCRIPTION OF THE INVENTION

### Example 1

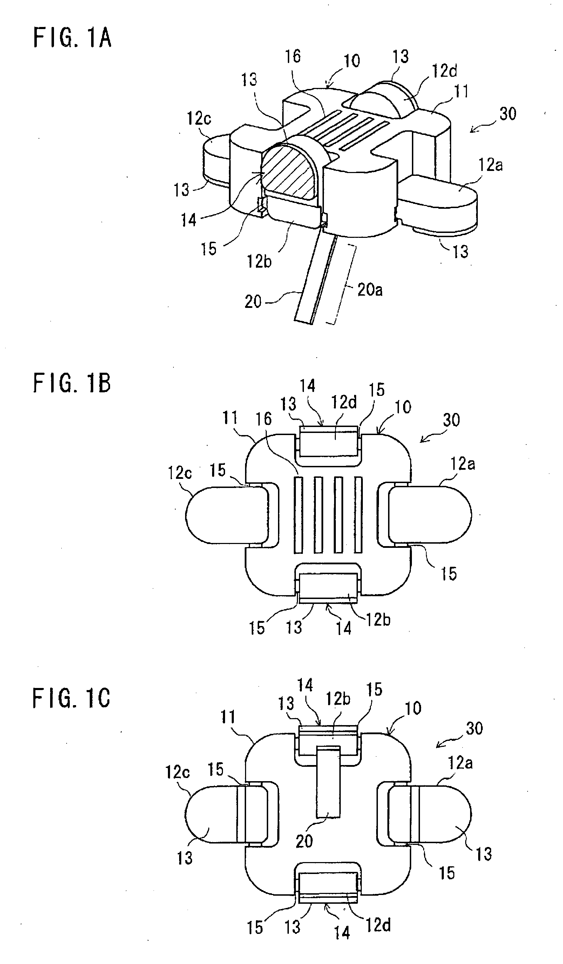

Hereinafter, a mount unit, a sensor unit, a measurement apparatus, and a sensor fixation method according to Example 1 of the present invention will be described with reference to FIGS. 1 to 4.

### Mount Unit and Sensor Unit

First, the configuration of a mount unit and a sensor unit according to Example 1 will be described with reference to FIGS.1. FIGS. 1A to 1C show the configuration of a mount unit and a sensor unit according to Example 1 of the present invention: FIG. 1A being a perspective view; FIG. 1B being a plan view; and FIG. 1C being a bottom view.

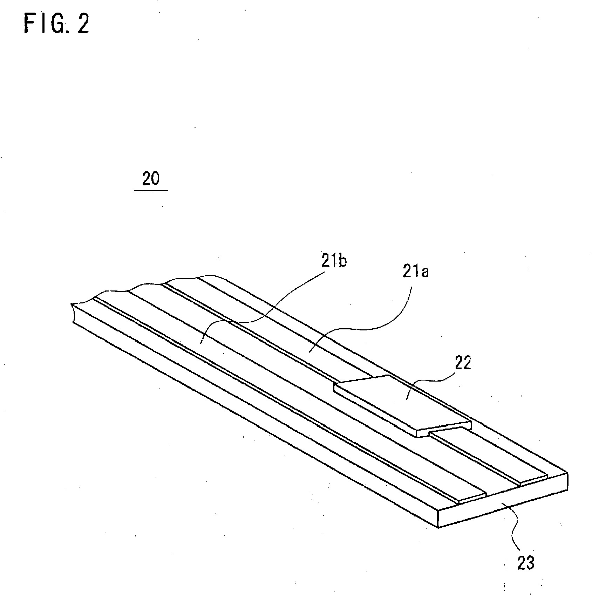

As shown in FIGS. 1A to 1C, in Example 1, a sensor unit 30 includes a mount unit 10 and a sensor 20 of which a portion 20a is placed under the skin. When the portion 20a is placed under the skin, the sensor 20 generates a signal according to numeric information relating to a substance contained in at least one of interstitial fluid and blood as will be described below with reference to FIG. 2. One example of such a substance is glucose. In this case, it is possible to identify numeric information relating to glucose, including, for example, glucose concentration, based on the signal from the sensor 20.

The mount unit 10 is used for fixing the sensor 20 to a living body, and includes a main body portion 11 and holding portions 12a to 12d. Of these, the main body portion 11 holds the placement of a portion of the sensor 20 under the skin of the living body. Specifically, in Example 1, the main body portion 11 holds the sensor 20 at a portion (not shown) of the sensor 20 that is not embedded under the skin. In Example 1, the main body portion 11 holds the sensor 20 such that the portion 20a of the sensor that is to be embedded under the skin projects from the living body side (see FIG. 1C) of the main body portion 11.

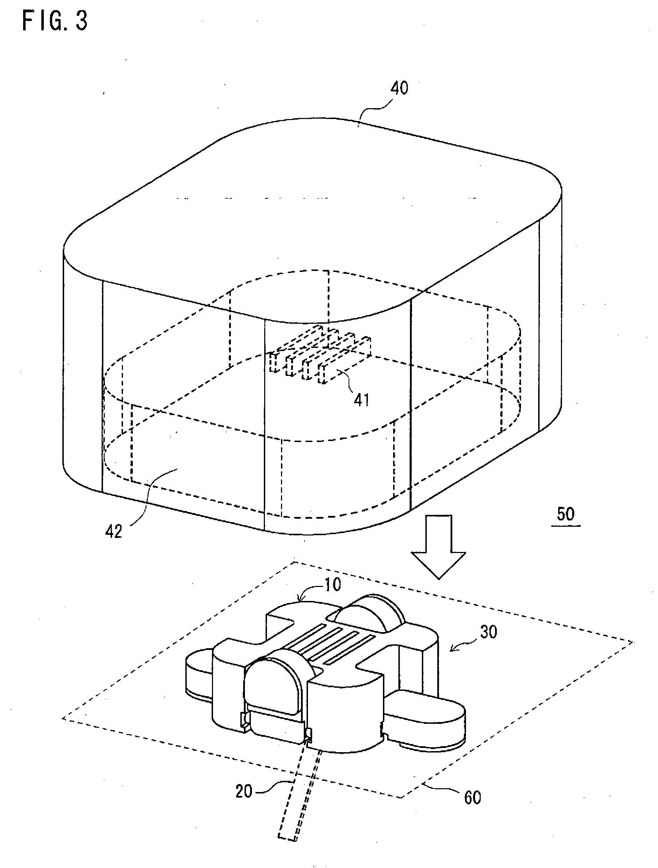

At this time, in the main body portion 11, electrical conduction is established between electrodes (see FIG. 2 described below) of the sensor 20 and electrodes 16 (see FIGS. 1A and 1B) exposed on the top face of the main body portion 11. Then, the electrodes of the sensor 20 are electrically connected to a control unit 40 (see FIG. 3 described below) via the electrodes 16.

The holding portions 12a to 12d are formed in an arm shape and attached to the main body portion 11. Also, each of the holding portions 12a to 12d includes an adhesive material layer 13 capable of adhering to the living body, and is movable so as to allow for selection between a state in which the adhesive material layer 13 is in contact with the living body and a state in which the adhesive material layer 13 is separate from the living body. Although the number of the holding portions in the example shown in FIGS. 1A to 1C is four, Example is not limited thereto. It suffices that the number of the holding portions is two or more.

Specifically, in Example 1, each of the holding portions 12a to 12d is attached so as to project from the side face of the main body portion 11 toward the periphery of the portion of the main body portion 11 on the living body side (see FIG. 1C). Also, each of the holding portions 12a to 12d is attached to the main body portion 11 via a hinge mechanism 15, and mobilization of the holding portions with the hinge mechanism 15 allows for selection between the state in which the adhesive material layer 13 is in contact with the living body and the state in which the adhesive material layer 13 is separate from the living body.

In the example shown FIGS. 1A to 1C, the holding portions 12a and 12c are in the state in which the adhesive material layer 13 is in contact with the living body. On the other hand, the holding portions 12b and 12d are in the state in which the adhesive material layer 13 is separate from the living body. Also, a separator film 14 is attached to the adhesive material layers 13 of the holding portions 12b and 12d in order to protect the adhesive material layers 13. In FIG. 1A, the separator films 14 are diagonally hatched. Note that illustration of the separator film 14 attached to the adhesive material layer 13 of the holding portion 12d is omitted.

One example of the usage of the mount unit 10 will now be described. For example, it is assumed that the mount unit 10 is adhered to the skin of a living body with the adhesive material layers 13 of the holding portions 12a and 12c, but the adhesive material layers 13 of both of these holding portions have been degraded. In this case, first, the separator films 14 attached to the adhesive material layers 13 of the holding portions 12b and 12d are detached. Next, the holding portions 12b and 12d are moved so as to be lifted down such that the adhesive material layers 13 of the holding portions 12b and 12d are adhered to the living body. Thereafter, by moving the holding portions 12a and 12c so as to be lifted up, it is possible to detach the degraded adhesive material layers 13 from the living body, while keeping the sensor 20 fixed. In this manner, according to Example 1, it is possible to replace the old adhesive material layers 13, while keeping the sensor 20 fixed.

Furthermore, in Example 1, any material having viscosity in two opposing directions may be used as the adhesive material layer 13. For example, the adhesive material layer 13 may be a layer formed only of an adhesive material, or may be a double-sided adhesive tape.

Specific examples of the double-sided adhesive tape include a double-sided adhesive tape in which an adhesive material layer is provided on either side of a base material formed of non woven fabric or the like. Examples of the adhesive material used in a layer formed only of an adhesive material and a double-sided adhesive tape include a hydrogel-based adhesive material and a silicone-based adhesive material. Of these, a silicone-based adhesive material can recover its viscosity by surface cleaning, and therefore an adhesive material layer or a double-sided adhesive tape for which a silicone-baced adhesive material is used can be used repeatedly.

Further, an adhesive material that can be detached by application of heat or voltage (for example, see JP 2010-037354A) can be used as the adhesive material for forming the adhesive material layers 13 in Example 1. By forming the adhesive material layers 13 with such an adhesive material, the adhesion between each of the holding portions and the skin can be selectively performed. Also, in this case, application of heat or voltage to the adhesive material layers 13 can be performed with a power source (not shown) of the mount unit.

### Sensor

The configuration of the sensor 20 will now be described with reference to FIG. 2 as well as FIGS. 1. FIG. 2 is a perspective view showing a tip portion of a sensor used in Example 1 of the present invention. The following description is given based on the assumption that the substance detected by the sensor 20 is glucose and the sensor 20 is a glucose sensor.

As shown in FIGS. 1 and 2, the sensor 20 is formed in the shape of an elongated band. The sensor 20 is brought into a state in which the portion 20a on the tip side is placed under the skin by an embedded device (not shown). Such a state of the sensor 20 can also be referred to as a state in which the sensor 20 is implanted in the skin.

As shown in FIG. 2, the sensor 20 includes an insulating, flexible substrate 23. There is no particular limitation with respect to the material for forming the substrate 23. However, for example, thermoplastic resins such as polyethylene terephthalate (PET), polypropylene (PP) and polyethylene (PE) and thermosetting resins such as polyimide resin and epoxy resin can be used as the material for forming the substrate 23 because these resins have little affect on the human body The shape of the substrate 23 may be, but is not particularly limited to, sharp-edged in order to facilitate the penetration into the skin of the living body.

In addition to the substrate 23, the sensor 20 includes a pair of electrodes 21a and 21b and a portion (enzyme reagent layer) 22 on which the glucose oxidoreductase is disposed. The electrodes 21a and 21b are used for applying voltage to the enzyme reagent layer 22. The electrodes 21a and 21b are formed on the surface of the substrate 23 in the longitudinal direction of the substrate 23, and also function as wiring. Additionally, the electrodes 21a and 21b may be formed by evaporation, screen printing, or the like, using, for example, a conductive material such as noncorrosive metal or carbon ink.

In the example shown in FIG. 2, the enzyme reagent layer 22 is formed by immobilizing glucose oxidoreductase on the electrode 21a. In this case, the electrode 21a functions as a working electrode. Glucose oxidoreductase has the function of detecting, on an electrode, a product resulting from the reaction with glucose (substrate) contained in interstitial fluid or blood, and the function of transferring electrons produced by the reaction to the electrode directly or via a mediator such as metal complex. Accordingly, upon application of voltage acaross the electrodes 21a and 21b, electrons produced by the catalyst reaction of the enzyme can be detected on electrode 21a according to the amount of glucose reacted in the reaction.

Examples of glucose oxidoreductase that can be used in Example 1 include glucose oxidase (GOD) and glucose dehydrogenase (GDH). Examples of the method for immobilizing glucose oxidoreductase include various known methods, including, for example, cross-linking using glutaraldehyde.

With this configuration, the value of the electric current flowing through the electrodes 21a and 21b changes according to the glucose concentration, and therefore the glucose concentration can be identified by measuring the current. In Example 1, the current flowing through the electrodes 21a and 21b corresponds to "signal dependent on numeric information relating to a substance".

### Measurement Apparatus

Next, the configuration of a measurement apparatus according to Example 1 will be described with reference to FIG. 3. FIG. 3 is a perspective view showing a measurement apparatus according to Example 1 of the present invention. A measurement apparatus 50 shown in FIG. 3 is an apparatus that measures numeric information relating to a substance contained in at least one of interstitial fluid and blood that are under the skin of a living body. As previously mentioned in the description in relation to FIGS. 1 and 2, one example of such a substance is glucose. In this case, one example of the numeric information is glucose concentration.

As shown in FIG. 3, the measurement apparatus 50 includes the sensor unit 30 shown in FIGS. 1A to 1C and a control unit 40. In the example shown in FIG. 3, the sensor unit 30 is disposed on the skin of a living body 60, and the tip portion 20a of the sensor 20 is placed under the skin of the living body 60.

The control unit 40 includes a recess 42 for housing the sensor unit 30 and electrodes 41. When the control unit 40 is disposed on the sensor unit 30 to house the sensor unit 30 in the recess 42, the electrodes 41 are connected to the electrodes 16 of the mount unit 10 and are consequently also connected to the electrodes 21a and 21b (see FIG. 2) of the sensor 20.

Further, the control unit 40 has the function of receiving a signal generated by the sensor 20 and executing the processing including digital signal processing on the received signal. Examples of the processing as mentioned herein include digital signal processing such as signal amplification processing and A/D conversion processing, and transmission processing. Specifically, the control unit 40 applies voltage to the electrodes 21a and 21b (see FIG. 2) of the sensor via the electrodes 16 of the mount unit 10. At the same time, the control unit 40 monitors the value of the current flowing through the electrodes 21a and 21b. Then, the control unit 40 generates, by digital processing, a digital signal identifying the level of the current value.

Thereafter, the control unit 40 can transmit the generated digital signal to an external measuring device via wired or wireless transmission. As with a conventional measuring device, this measuring device calculates a specific glucose concentration based on the received digital signal and displays the calculated value on a display screen or the like. Note that the control unit 40 itself may perform the glucose concentration calculation and the like in this embodiment.

### Sensor Fixation Method

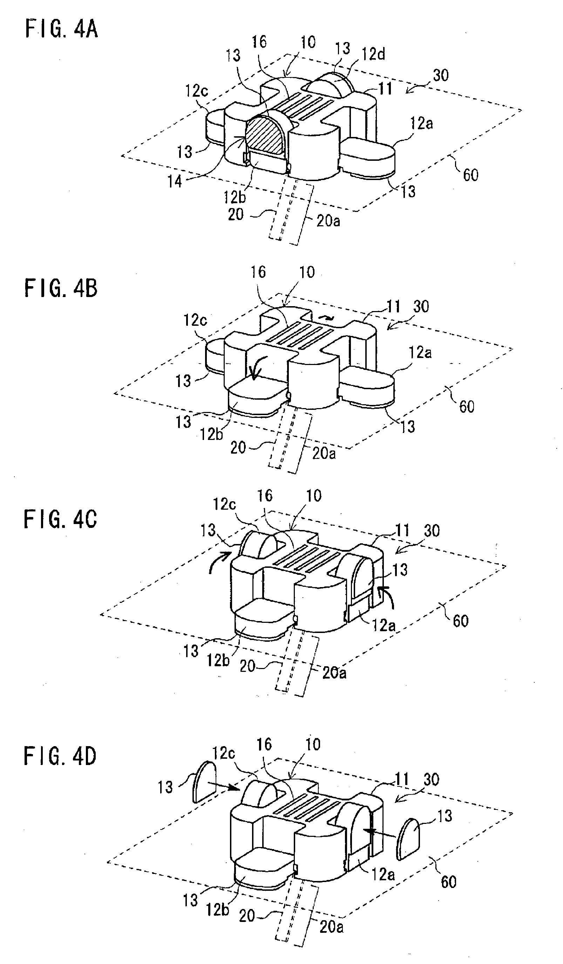

Next, a sensor fixation method according to Example 1 of the present invention will be described with reference to FIGS. 4A to 4D. FIGS. 4A to 4D show a series of main steps constituting a sensor fixation method according to Example 1. of the present invention.

In the example shown in FIGS. 4A and 4B, the sensor unit 30 is disposed on the skin of the living body 60, and the tip portion 20a of the sensor 20 is placed under the skin of the living body 60. Also, the mount unit 10 constituting the sensor unit 30 is adhered to the skin of the living body 60 with the adhesive material layers 13 of the holding portions 12a and 12c, but the adhesive material layers 13 of both of these holding portions have been degraded.

The following is a description of a method for fixing the sensor 20 to the living body 60 in the above-mentioned case, while replacing a degraded adhesive material layer 13 with a new adhesive material layer 13. In the following description, reference is made to FIGS.1 to 3 as needed.

As shown in FIG. 4A, the adhesive material layers 13 of the holding portions 12b and 12d are unused, and therefore the separator films 14 are attached to the adhesive material layers 13. The separator films 14 are diagonally hatched in FIG. 4A as well. However, illustration of the separator film 14 attached to the adhesive material layer 13 of the holding portion 12d is omitted.

Next, after the separator films 14 are detached from the adhesive material layers 13, the holding portions 12b and 12d are lifted down such that the adhesive materials layers 13 of the holding portions 12b and 12d are adhered to the living body 60 as shown in FIG. 4B. Thereafter, as shown in FIG. 4C, the holding portions 12a and 12c are lifted up, and the degraded adhesive material layers 13 are detached from the living body 60.

Next, as shown in FIG. 4D, the degraded adhesive material layers 13 are removed from the holding portions 12a and 12c. Then, new adhesive material layers 13 are applied to the holding portions 12a and 12c. The new adhesive material layers 13 are used when the adhesive material layers 13 of the holding portions 12b and 12d have been degraded.

In this manner, according to Embodiment 1, it is possible to detach a degraded adhesive material layer 13 from the living body 60, while keeping the sensor 20 fixed. Furthermore, it is possible to replace a degraded adhesive material layer 13 with a new adhesive material layer, while keeping the sensor 20 fixed.

Although the mount unit 10 is fixed using only two of the four holding portions in the example shown in FIGS. 4A to 4D, Example 1 is not limited thereto. In Embodiment 1, the mount unit 10 may be fixed using all of the holding portions 12a to 12d. In this case, with at least only one holding portion lifted down, the lifting-up of other holding portions and the replacement of the adhesive material layers 13 may be performed. Thereafter, the holding portions whose adhesive material layers 13 have been replaced may be lifted down, and the lifting-up of the remaining holding portions and the replacement of the adhesive material layers 13 may be performed. Accordingly, it is possible to replace all of the degraded adhesive material layer 13 with new adhesive material layers 13, while keeping the sensor 20 fixed.

### Example 2

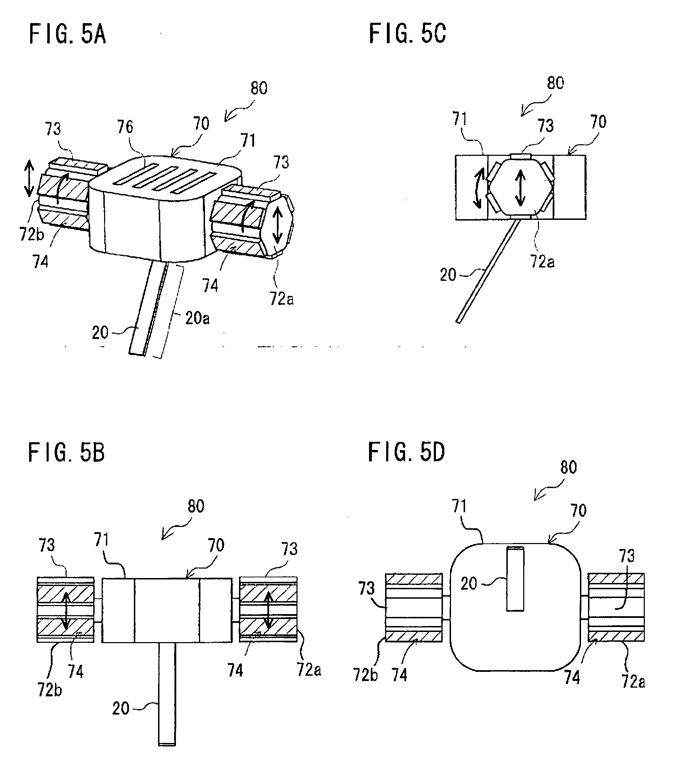

Next, a mount unit, a sensor unit, a measurement apparatus, and a sensor fixation method according to Embodiment 2 of the present invention will be described with reference to FIGS. 5 to 7.

### Mount unit, Sensor Unit, and Measurement Apparatus

First, the configuration of a mount unit, a sensor unit, and a measurement apparatus according to Example 2 will be described with reference to FIGS. 5. FIGS. 5A to 5D show the configuration of a mount unit and a sensor unit according to Embodiment 2 of the present invention: FIG. 5A being a perspective view; FIG. 5B being a front view; FIG. 5C being a side view; and FIG. 5D being a bottom view.

As shown in FIGS. 5A to 5D, a sensor unit 80 includes a mount unit 70 and a sensor 20 in Example 2. The sensor 20 is the same as the sensor described in Example 1 with reference to FIG. 2. The mount unit 70 includes a main body portions 71. The main body portion 71 holds a portion (not shown) of the sensor 20 that is not embedded under the skin, as with the main body portion 11 of the mount unit 10 shown in FIGS.1.

Although the mount unit 70 includes holding portions as with the mount unit 10 shown in FIGS.1, the holding portions of the mount unit 70 in Embodiment 2 are different in configuration from those in Example 1. The following description is focused on the difference between Example 2 and Example 1.

As shown in FIGS. 5A to 5D, the sensor unit 70 includes holding portions 72a and 72b unlike the mount unit 10 shown in FIGS. 1. Each of the holding portions 72a and 72b is attached to the main body portion 71 so as to be movable in a direction toward the living body and a direction away from the living body in the projecting state (see FIGS. 5B and 5C).

In other words, each of the holding portions 72a and 72b is capable of moving vertically in the thickness direction of the main body portion 71. Then, movement of each of the holding portions 72a and 72b allows for selection between a state in which the adhesive material layer 73 is in contact with the living body and a state in which the adhesive material layer 73 is separate from the living body:

Each of the holding portions 72a and 72b has the shape of a roller and is attached rotatable to the main body portion 71. Specifically, each of the holding portions 72a and 72b can also rotate about its axis in the projecting direction, that is, its axis expending from the portion attached to the main body portion 71 toward the tip end portion as the rotation axis (see FIGS. 5A and 5C).

Further, a plurality of adhesive material layers 73 are provided on the outer face of each of the holding portions 72a and 72b in the direction of rotation. With this configuration, in Example 2, it is possible to replace a degraded adhesive material layer with a new adhesive material layer 73 by simply rotating the holding portion 72a or 72b as will be described below.

Note that a separator film 74 is attached to unused adhesive material layers 73 for protecting the adhesive material layers 73. In FIGS. 5A, 5B and 5D, the separator films 74 are diagonally hatched.

Although only two holding portions are shown in the example shown in FIGS. 5A to 5D, it suffices that the number of the holding portions is two or more in Example 2 as well. For example, the number of the holding portions may be three or more. Further, in the case where the number of holding portions are three or more in Example 2, the mount unit 70 may be fixed using only some of the holding portions (for example, two holding portions), or may be fixed using all of the holding portions.

In Example 2 as well, the measurement apparatus includes a sensor unit 80 and a control unit as with Example 1. The control unit may be the same as the control unit 40 shown in FIG. 3 in Example 1. In this case, the measurement apparatus according to Example 2 can be configured by attaching the control unit to the sensor unit 80.

### Sensor Fixation Method

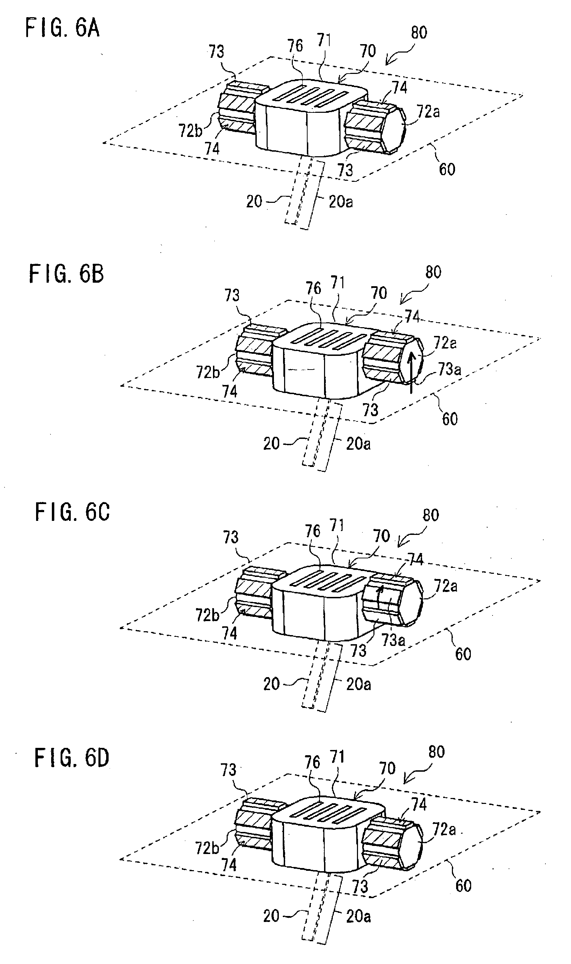

Next, a sensor fixation method according to Example 2 of the present invention will be described with reference to FIGS. 6A to 6D. FIGS. 6A to 6D show a series of main steps constituting a sensor fixation method according to Example 2 of the present invention.

In the example shown in FIGS. 6A to 6D, the sensor unit 80 is disposed on the skin of the living body 60, and the tip portion 20a of the sensor 20 is placed under the skin of the living body 60. Also, the mount unit 70 constituting the sensor unit 80 is adhered to the skin of the living body 60 with one of the adhesive material layers 73 of each of the holding portions 72a and 72b, but the adhesive material layers 73 in use have been degraded.

The following is a description of a method for fixing the sensor 20 to the living body 60 in the above-mentioned case, while replacing a degraded adhesive material layer 73a (see FIGS. 6B and (c)) with a new adhesive material layer 73. In the following description, reference is made to FIGS. 5 as needed.

As shown in FIG. 6A, separator films 74 are attached to unused adhesive material layers 73 in the holding portions 72a and 72b. The separator films 74 are diagonally hatched in FIGS. 6A to 6D as well.

Next, as shown in FIG. 6B, only the holding portion 72a is moved in the direction away from the living body 60, and the degraded adhesive material layer 73a of the holding portion 72a is detached from the living body. At this time, the holding portion 72b is left as is, and therefore, the mount unit 70 is kept fixed to the living body 60 with the adhesive material layer 73 of the holding portion 72b.

Net, as shown in FIG. 6C, the holding portion 72a is rotated, and the separator film 74 of an unused adhesive material layer 73 is detached. Thereafter, the holding portion 72a is further rotated, and thereby the adhesive material layer 73 from which the separator film 74 has been detached is faced toward the living body 60 instead of the degraded adhesive material layer 73a.

Next, as shown in FIG. 6D, the holding portion 72a is moved in the direction toward the living body 60, and thereby the adhesive material layer 73 from which the separator film 74 has been detached in the step shown in FIG. 6C is adhered to the living body 60. By the steps shown in FIGS. 6A to 6D, adhesion to the living body 60 is achieved by the new adhesive material layer 73 replacing the degraded adhesive material layer 73a in the holding portion 72a.

After performing the steps shown in FIGS. 6A to 6D for the holding portion 72b, adhesion to the living body 60 is achieved with the new adhesive material layer 73 replacing the degraded adhesive material layer in the holding portion 72b as well.

In this manner, according to Example 2 as well, it is possible to detach the degraded adhesive material layer 73a (see FIGS. 6B and 6C) from the living body 60, while keeping the sensor 20 fixed, as with Embodiment 1. Furthermore, it is possible to replace the degraded adhesive material layer 73a with a new adhesive material layer 73, while keeping the sensor 20 fixed. Furthermore, according to Example 2, it is possible to replace the degraded adhesive material layer 73a with a new adhesive material layer 73 by simply rotating the holding portion 72a or 72b.

### Modification

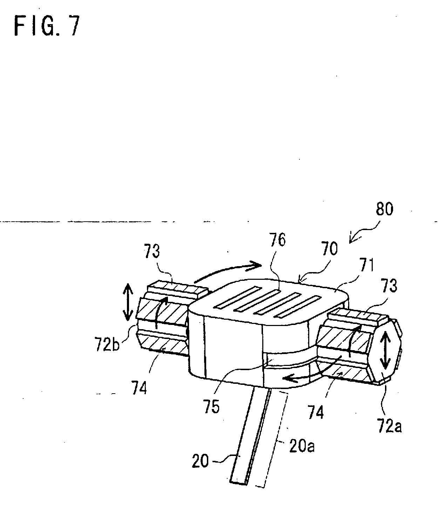

Here, a modification of the mount unit according to Example 2 will be described with reference to FIG. 7. FIG. 7 is a perspective view showing a modification of the mount unit according to Example 2 of the present invention.

As shown in FIG. 7, in this modification, grooves 75 that permit the horizontal movement of the holding portions are provided on the side face of the main body portion 71. In FIG. 7, only the groove 75 corresponding to the holding portion 72a is shown.

In this manner, this modification includes a mechanism that can change the contact position of at least one holding portion that is in contact with the living body, in a state where the position of the sensor 20 is held. This makes it possible to change the portion of the skin that is in contact with the adhesive material layer 73, without changing the position of the sensor 20. Accordingly, it is possible to suppress a skin irritation resulting from a long period of contact between the skin and the adhesive material layer 73.

### Example 3

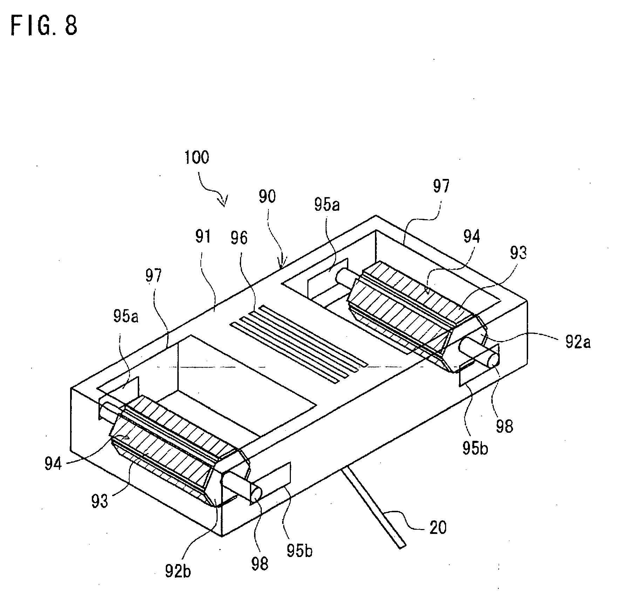

Next, a mount unit, a sensor unit, a measurement apparatus, and a sensor fixation method according to Example 3 of the present invention will be described with reference to FIGS. 8 to 10.

### Mount unit, Sensor unit, and Measurement Apparatus

First, the configuration of a mount unit, a sensor unit, and a measurement apparatus according to Example 3 will be described with reference to FIG. 8. FIG. 8 shows the configuration of a mount unit according to Example 3 of the present invention.

As shown in FIG. 8, in Example 3, a sensor unit 100 includes a mount unit 90 and a sensor 20. The sensor 20 is the same as the sensor described in Example 1 with reference to FIG. 2.

Also, the mount unit 90 includes a main body portion 91 to which holding portions 92a and 92b are attached. Further, as with the holding portions 72a and 72b shown in FIGS. 5A to 5D in Embodiment 2, the holding portions 92a and 92b have the shape of a roller and are attached rotatably to the main body portion 91.

Further, in Example 3 as well, a plurality of adhesive material layers 93 are provided on the outer face of each of the holding portions 92a and 92b in the direction of rotation as with Example 2. Accordingly it is possible to replace a degraded adhesive material layer with a new adhesive material layer 93 by simply rotating the holding portion 92a or 92b. Note that a separator film 94 is attached to unused adhesive material layers 93 for protecting the adhesive material layer 93. In FIG. 8, the separator films 94 are diagonally hatched.

However, unlike in Example 2, the holding portions 92a and 92b are attached inside the main body portion 91 in Example 3. Note that, for the sake of illustration, the part of the holding portions 92a and 92b that is actually hidden behind the main body portion 91 and thus cannot be visually observed is also shown in FIG. 8.

Specifically, as shown in FIG. 8, the main body portion 91 includes, on opposing side faces thereof, two sets of pairs of grooves 95a and 95b. Each of the holding portions 92a and 92b is held between a groove 95a and a groove 95b in a state in which it is sandwiched between the groove 95a and the groove 95b.

Sandwiching between the grooves 95a and 95b is achieved by catching, on the respective grooves, both ends of a shaft 98 passing through the center of each of the holding portions. Additionally, each of the grooves is formed in a rectangular shape in the horizontal direction of the main body portion 91, and the width of the grooves is set: to be greater than the diameter of the shaft 98.

This enables the holding portions 92a and 92b to move vertically (in the thickness direction of the main body portion) and horizontally. Further, the main body portion 91 is provided with an opening 97 for each holding portion for allowing the operator to manually move the holding portions 92a and 92b. That is, the mount unit 90 includes a mechanism that can change the contact position of at least one holding portion that is in contact with the living body, in a state in which the position of the sensor 20 is held.

Although only two holding portions are shown in the example shown in FIG. 8, it suffices that the number of the holding portions is two or more in Example 3 as well. For example, the number of the holding portions may be three or more. Further, in the case where the number of holding portions are three or more in Example 3, the mount unit 90 may be fixed using only some of the holding portions (for example, two holding portions), or may be fixed using all of the holding portions.

In Example 3 as well, the measurement apparatus includes a sensor unit 90 and a control unit as with Example 1. The control unit may be the same as the control unit 40 shown in FIG. 3 in Example 1. In this case, the measurement apparatus according to Example 3 can be configured by attaching the control unit to the sensor unit 90.

### Sensor Fixation Method

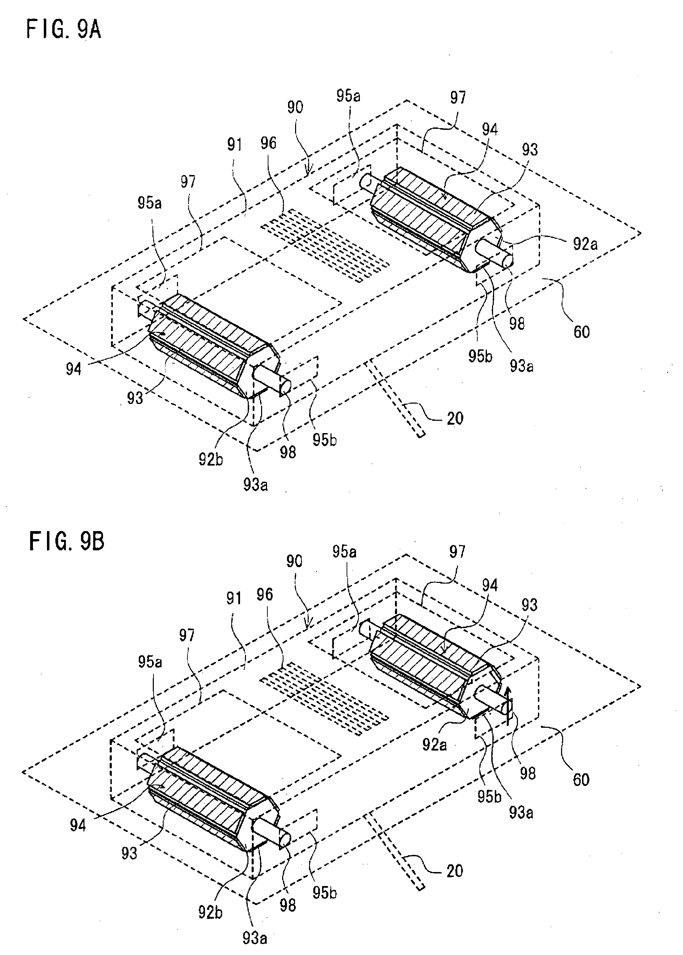

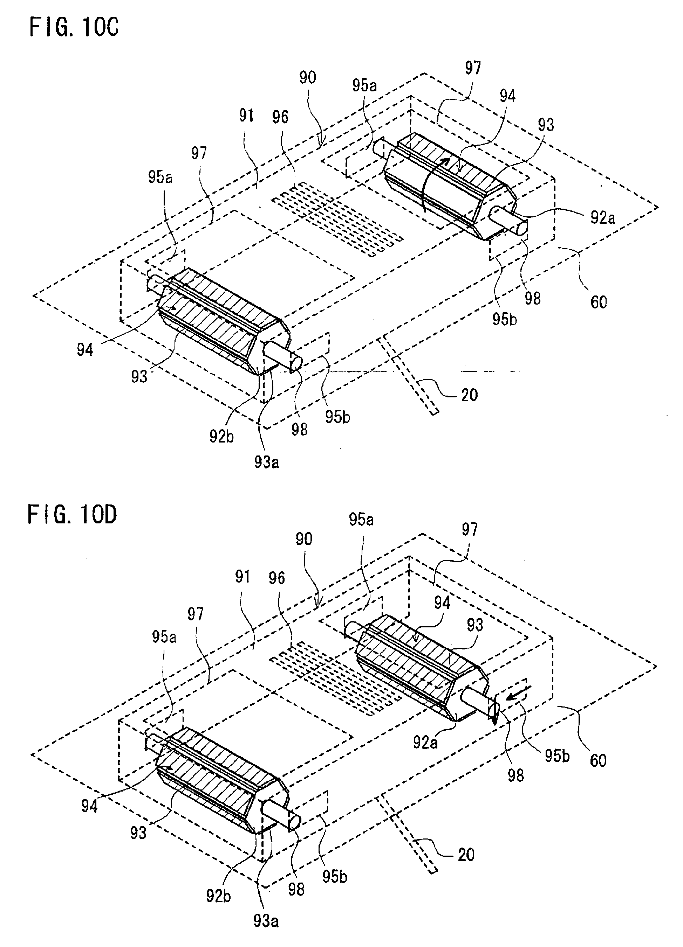

Next, a sensor fixation method according to Example 3 of the present invention will be described with reference to FIGS. 9A to 10D. FIGS. 9A an 9B show a series of main steps constituting a sensor fixation method according to Example 3 of the present invention. FIGS. 10C and 10D show a series of main steps constituting a sensor fixation method according to Example 3 of the present invention, which are executed after execution of the step shown in FIG. 9B.

In the example shown in FIGS. 9A to 10D, a sensor unit 100 is disposed on the skin of a living body 60, and the tip portion of the sensor 20 is placed under the skin of the living body 60.

An shown in FIG. 9A, the mount unit 90 constituting the sensor unit 100 is adhered to the skin of the living body 60 with one of the adhesive material layers 93 of each of the holding portions 92a and 92b. However, the adhesive material layers 93a in use have been degraded and needs to be replaced. On the other hand, a separator film 94 is attached to unused adhesive material layers 93 of the holding portions 92a and 92b.

Next, as shown in FIG. 9B, only the holding portion 92a is moved in the direction away from the living body 60, i.e., moved upward, and the degraded adhesive material layer 93a of the holding portion 92a is detached from the living body At this time, the holding portion 92b is left as is, and therefore, the mount unit 90 is kept fixed to the living body 60 with the adhesive material layer 93a of the holding portion 92b.

Next, as shown in FIG. 10C, the holding portion 92a is rotated, and the separator film 94 of an unused adhesive material layer 93 is detached. Thereafter, the holding portion 92a is further rotated, and thereby the adhesive material layer 93 from which the separator film 94 has been detached is faced toward the living body 60 instead of the degraded adhesive material layer 93a.

Next, as shown in FIG. 10D, the holding portion 92a is moved horizontally. Thereafter, the holding portion 92a is moved in the direction toward the living body 60, i.e., downward, before the adhesive material layer 93 from which the separator film 94 has been detached in the step shown in FIG. 10C is adhered to the living body 60. Thereafter the above-described upward mouvement, detachment of the separator film, rotation, horizontal movement, and adhesion by the new adhesive material layer are similarly performed for the holding portion 92b as well.

Consequently according to Example 3 as well, it is possible to detach the degraded adhesive material layer 93a (see FIG. 9A) from the living body 60 and replace the degraded adhesive material layer 93a with a new adhesive material layer 93, while keeping the sensor 20 fixed, as with Example 1.

Furthermore, according to Example 3, it is possible to change the portion of the skin that is in contact with the adhesive material layer 93 by horizontal movement of the holding portions 92a and 92b, and therefore it is possible to suppress a skin irritation resulting from a long period of contact between the skin and the adhesive material layer 93, as with the modification of Example 2. In addition, according to Embodiment 3, the holding portions 92a and 92b are housed inside the main body portion 91, and therefore the detachment of the adhesive material layer 93 from the living body 60 due to external force applied to the holding portions 92a and 92b is also suppressed.

### Example 4

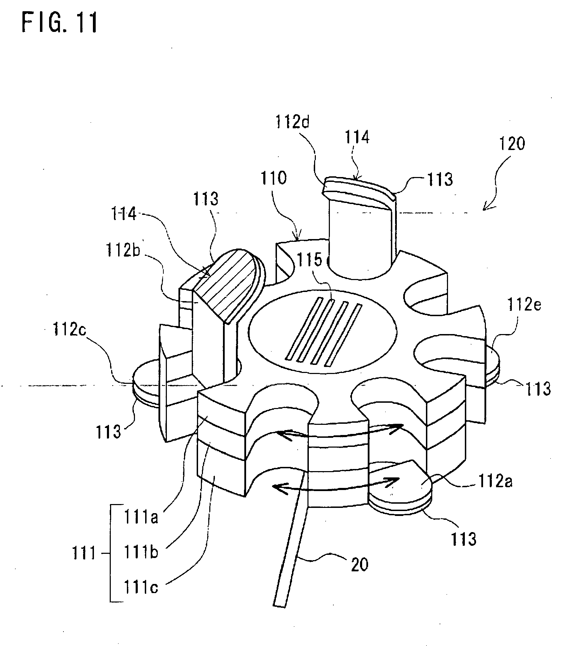

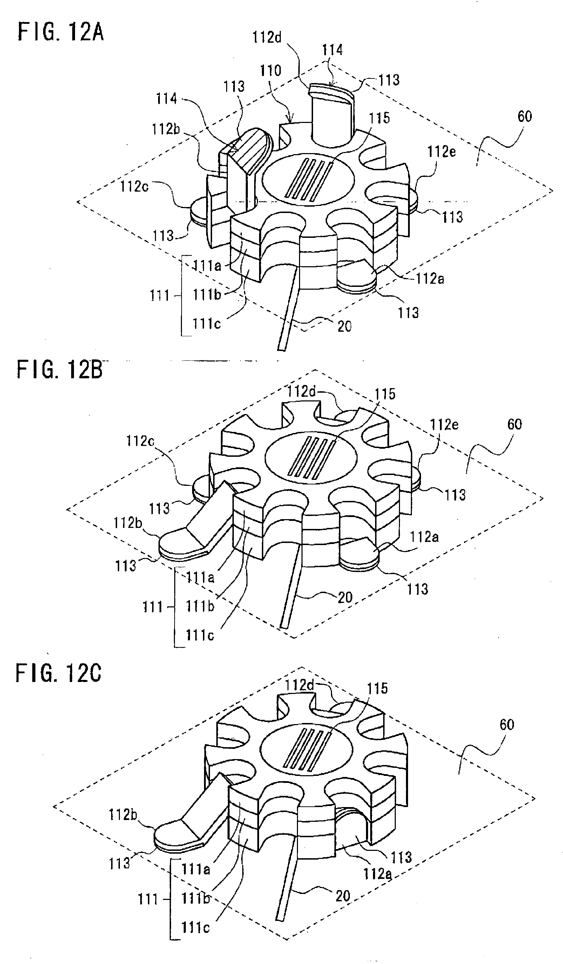

Next, a mount unit, a sensor unit, a measurement apparatus, and a sensor fixation method according to Example 4 of the present invention will be described with reference to FIGS. 11 and 12.

### Mount unit, Sensor unit, and Measurement Apparatus

First, the configuration of a mount unit, a sensor unit, and a measurement apparatus according to Example 4 will be described with reference to FIG. 11. FIG. 11 is a perspective view showing a mount unit according to Embodiment 4 of the present invention.

As shown in FIG. 11, a sensor unit 120 includes a mount unit 110 and a sensor 20 in Example 4. The sensor 20 is the same as the sensor described in Example 1 with reference to FIG. 2.

The mount unit 110 includes a main body portion 111 to which holding portions 112a to 112e are attached. As with the holding portions 12a to 12d shown in FIGS. 1 in Embodiment 1, the holding portions 112a to 112e are formed in an arm shape and attached to the side face of the main body portion 111 via a hinge mechanism (not shown in FIG. 11).

For each of the holding portions 112a to 112e as well, mobilization of the holding portions with the hinge mechanism 15 allows for selection between the state in which the adhesive material layer 113 is in contact with the living body and the state in which the adhesive material layer 113 is separate from the living body Further, an adhesive material layer 113 capable of adhering to the living body is provided on the face of each holding portion on the living body side.

In this manner, the mount unit 110 includes movable holding portions in Example 4 as with the mount unit 10 shown in FIGS. 1 in Example 1. However the mount unit 110 is different from the mount unit 10 in the following aspects.

In Example 4, the mount unit 110 includes a mechanism that can change the contact position of at least one holding portion that is in contact with the living body, in a state in which the position of the sensor 20 is held. Specifically, in Example 4, the main body portion 111 includes an upper portion 111a, a middle portion 111b, and a lower portion 111c. Of these, the upper portion 111a and the lower portion 111c are each attached to the middle portion 111b such that they are independently rotatable in the direction indicated by the arrows in FIG. 11. The sensor 20 and electrodes 115 are attached to the middle portion 111b and remain stationary even if the upper portion 111a and the lower portion 111c are rotated.

Of the holding portions, the holding portions 112a, 112c, and 112e are attached to the lower portion 111c. Also, the holding portions 112a, 112c, and 112e are formed so as not to prevent rotation of the upper portion 111a when they are separ ated from the living body (when they are lifted up).

On the other hand, the holding portions 112b and 112d are attached to the upper portion 111a. The holding portions 112b and 112d are formed so as not to prevent rotation of the lower portion 111c when they are brought into contact with the living body (when they are lifted down). In other words, the holding portions 112b and 112d are formed so as to come into contact with the living body when they are lifted down, at positions further removed than the holding portion 112a, 112c, an 112e, so as not to come into contact with the lower portion 111c.

In the example show in FIG. 11, the holding portions 112a, 112c, and 112e are in the state in which the adhesive material layers 113 are in contact with the living body. On the other hand, the holding portions 112b and 112d are in the state in which the adhesive material layers 113 are separate from the living body. Also, a separator film 114 is attached to the adhesive material layers 113 of the holding portions 112b and 112d for protecting the adhesive material layers 113. In FIG. 11, the separator films 114 are diagonally hatched. Note that illustration of the separator film 114 attached to the adhesive material layer 13 of the holding portion 112d is omitted.

### Sensor Fixation Method

Next, a sensor fixation method according to Example 4 of the present invention will be described with reference to FIGS. 12. FIGS. 12A to 12C show a series of main steps constituting a sensor fixation method according to Example 4 of the present invention.

In the example shown in FIGS. 12A to 12C, the sensor unit 120 is disposed on the skin of a living body 60, and the tip portion of the sensor 20 is placed under the skin of the living body 60.

As shown in FIG. 12A the mount unit 110 constituting the sensor unit 120 is adhered to the skin of the living body 60 with the adhesive material layers 113 of the holding portions 112a, 112c, and 112e. However, the adhesive material layers 113 of at least one of the holding portions 112a,112c, and 112e has been degraded and needs to be replaced. On the other hand, the adhesive material layers 113 of the holding portions 112b and 112d are unused, and therefore a separator film 114 is attached thereto.

Next, after the separator films 114 are detached from the adhesive material layers 113, the holding portions 112b and 112d are lifted down and the adhesive material layers 113 thereof are adhered to the living body 60 as shown in FIG. 12B. At this time, the position of contact between each of the holding portions 112b and 112d and the living body 60 can be selected by rotating the upper portion 111a.

Next, as shown in FIG. 12C, the holding portions 112a, 112c, and 112e are brought into the lifted state (the state in which they are separate from the living body 60). Then, in this state, the degraded adhesive material layer 113 is replaced.

Thereafter, in the case where the adhesive material layers 113 of the holding portions 112b and 112d have been degraded, the position of the contact between each of the holding portions 112a, 112c, and 112e and the living body 60 is selected by rotating the lower portion 111c, and then the holding portions 112a, 112c, and 112e are brought into contact with the living body 60 again. Then, the holding portions 112b and 112d are brought into the lifted state.

In this manner, according to Example 4, it is possible to detach a degraded adhesive material layer 113 from the living body 60, while keeping the sensor 20 fixed, as with Example 1. Furthermore, it is also possible to replace a degraded adhesive material layer 113 with a new adhesive material layer, while keeping the sensor 20 fixed.

Furthermore, according to Example 4, the upper portion 111a and the lower portion 111c can be rotated independently, thus making it possible to change the portion of the living body that is in contact with the adhesive material layer 113, while keeping the sensor 20 fixed. Therefore, according to Example 4 as well, it is possible to suppress a skin irritation resulting from a long period of contact between the skin and the adhesive material layer 113, as with the modification of Example 2 and Example 3.

### Supplementary note 1

As described above, according to the present invention, in the case where adhesive tape or the like is used for fixing a mount of an embedded sensor to a living body it is possible to easily replace the adhesive tape while preventing external force from being applied to the sensor. The present device and method is particularly useful in the filed of CGM, in which such a demand exists.

## Claims

1. A mount unit(10) comprising:
a main body portion(11) adapted to hold a portion of a sensor(20) under the skin of a living body(60); and
two or more holding portions(12a,12b,12c,12d) attached to the main body portion(11).
wherein each of the two or more holding portions(12a,12b,12c,12d) comprises an adhesive material layer(13) capable of adhering to the living body(60) and is movable so as to allow for selection between a state in which the adhesive material layer(13) is in contact with the living body(60) and a state in which the adhesive material laver(13) is separate from the living bodv(60),
wherein the main body portion (11) comprises a mechanism that can change a contact position of at least one of the holding portions (12a. 12b, 12c, 12d) that is in contact with the living body (60), in a state in which the position of the sensor (20) is held.

2. The mount unit(10) according to claim 1.
wherein each of the two or more holding portions(12a,12b,12c,12d) is attached to the main body portion(11) via a hinge mechanism(15), and
mobilization of the holding portions(12a,12b,12c,12d) with the hinge mechanism(15) allows for selection between a state in which the adhesive material laver(13) is in contact with the living body(60) and a state in which the adhesive material layer(13) is separate from the living body(60).

3. The mount unit (10) according to claim 1.
wherein each of the two or more holding portions (12a, 12b, 12c, 12d) is attached to the main body portion (11) so as to be movable in a direction toward the living body (60) and a direction away from the living body (60), and
movement of the holding portions (12a, 12b, 12c, 12d) allows for selection between a state in which the adhesive material layer (13) is in contact with the living body (60) and a state in which the adhesive material layer (13) is separate from the living body (60).

4. The mount unit (10) according to claim 3, wherein each of the two or more holding portions (12a, 12b, 12c, 12d) is attached to the main body portion (11) so as to further be rotatable with respect to the main body portion (11).

5. The mount unit (10) according to claim 4, wherein a plurality of the adhesive material layers (13) are provided on an outer face of each of the two or more holding portions (12a, 12b, 12c, 12d) in the direction of rotation.

6. A sensor unit(30) comprising: a sensor(20) of which a portion can be placed under the skin of a living body(60); and the mount unit(10) according to claim 1 for fixing the sensor(20) to the living body(60).

7. A measurement apparatus(50) for measuring numeric information relating to a substance contained in at least one of interstitial fluid and blood that are under the skin of a living body(60), the apparatus(50) comprising:
a sensor(20) of which a portion can be placed under the skin and that is configured to generate a signal dependent on the numeric information;
the mount unit(10) according to claim 1 for fixing the sensor(20) to the living body(60); and
a control unit(40) adapted to receive the signal generated by the sensor(20) and to execute processing that includes digital signal processing on the signal.

8. The measurement apparatus(50) according to claim 7, wherein the control unt(40) is adapted to execute transmission processing for transmitting the signal that has been subjected to digital signal processing to an external measuring device.

9. A sensor fixation method for fixing, to a living body (60), a sensor (20) of which a portion can be placed under the skin of the living body (60), the method comprising the steps of:
(a) using a mount unit (10) that comprises a main body portion (11) and two or more holding portions (12a, 12b, 12c, 12d) attached to the main body portion (11), each of the two or more holding portions (12a, 12b, 12c, 12d) comprising an adhesive material layer (13) capable of adhering to the living body (60) and attached to the main body portion (11) so as to allow for selection between a state in which the adhesive material layers (13) are in contact with the living body (60) and a state in which the adhesive material layers (13) are separate from the living body (60), and holding the sensor (20) by the main body portion (11) of the mount unit (10);
(b) fixing the mount unit (10) to the living body (60) by bringing the adhesive material layer (13) of at least one of the two or more holding portions (12a, 12b. 12c, 12d) of the mount unit (10) into contact with the living body (60); and,
(c) in a case where any of the adhesive material layers (13) of the two or more holding portions (12a, 12b, 12c, 12d) needs to be replaced, moving the holding portion (12a, 12b, 12c, 12d) including the adhesive material layer (13) that needs to be replaced, in a state in which the adhesive material layer (13) of at least one of the holding portions (12a, 12b, 12c, 12d) is in contact with the living body (60), and detaching the adhesive material layer (13) that needs to be replaced from the living body (60).

10. The sensor fixation method according to claim 9.
wherein each of the two or more holding portions (12a. 12b, 12c, 12d) is attached to the main body portion (11) via a hinge mechanism (15),
mobilization of the holding portions (12a, 12b, 12c, 12d) with the hinge mechanism (15) allows for selection between a state in which the adhesive material layer (13) is in contact with the living body (60) and a state in which the adhesive material layer (13) is separate from the living body (60), and,
in step (c), the adhesive material layer (13) that needs to be replaced is detached from the living body (60) by mobilization of the holding portions (12a, 12b, 12c, 12d) with the hinge mechanism (15).

11. The sensor fixation method according to claim 9,
wherein each of the two or more holding portions (12a, 12b, 12c, 12d) is attached to the main body portion (11) so as to be movable in a direction toward the living body (60) and a direction away from the living body (60),
movement of the holding portions (12a, 12b, 12c, 12d) allows for selection between a state in which the adhesive material layer (13) is in contact with the living body (60) and a state in which the adhesive material layer (13) is separate from the living body (60), and,
in step (c), the adhesive material layer (13) that needs to be replaced is detached from the living body (60) by movement of the holding portions (12a, 12b, 12c, 12d).

12. The sensor fixation method according to claim 11, wherein each of the two or more holding portions (12a, 12b, 12c, 12d) is attached to the main body portion (11) so as to be rotatable with respect to the main body portion (11).

13. The sensor Fixation method according to claim 12.
wherein a plurality of the adhesive material layers (13) are provided on an outer face of each of the two or more holding portions (12a, 12b, 12c, 12d) in the direction of rotation, and,
in step (c), a new adhesive material layer (13) replacing the adhesive material layer (13) that needs to be replaced is faced toward the living body (60) by rotation of the holding portions (12a, 12b, 12c, 12d), and thereafter the new adhesive material layer (13) is adhered to the living body (60) by movement of the holding portions (12a, 12b, 12c, 12d).

## Patentansprüche

1. Montageeinheit (10), die Folgendes umfasst:
einen Hauptkörperteil (11) zum Halten eines Teils eines Sensors (20) unter der Haut eines lebenden Körpers (60); und
zwei oder mehr Halteteile (12a, 12b, 12c, 12d), die an dem Hauptkörperteil (11) angebracht sind,
wobei jeder der zwei oder mehr Halteteile (12a, 12b, 12c, 12d) eine Klebstoffschicht (13) aufweist, die an dem lebenden Körper (60) haften kann und beweglich ist, um eine Auswahl zwischen einem Zustand, in dem die Klebstoffschicht (13) mit dem lebenden Körper (60) in Kontakt ist, und einem Zustand zu ermöglichen, in dem die Klebstoffschicht (13) von dem lebenden Körper (60) getrennt ist,
wobei der Hauptkörperteil (11) einen Mechanismus umfasst, der eine Kontaktposition von wenigstens einem der Halteteile (12a, 12b, 12c, 12d), die mit dem lebenden Körper (60) in Kontakt sind, in einen Zustand ändern kann, in dem die Position des Sensors (20) gehalten wird.

2. Montageeinheit (10) nach Anspruch 1,
wobei jeder der zwei oder mehr Halteteile (12a, 12b, 12c, 12d) über einen Gelenkmechanismus (15) an dem Hauptkörperteil (11) angebracht ist, und
eine Mobilisierung der Halteteile (12a, 12b, 12c, 12d) mit dem Gelenkmechanismus (15) das Auswählen zwischen einem Zustand, in dem die Klebstoffschicht (13) mit dem lebenden Körper (60) in Kontakt ist, und einem Zustand ermöglicht, in dem die Klebstoffschicht (13) von dem lebenden Körper (60) getrennt ist.

3. Montageeinheit (10) nach Anspruch 1,
wobei jeder der zwei oder mehr Halteteile (12a, 12b, 12c, 12d) so am Hauptkörperteil (11) angebracht ist, dass er in einer Richtung zu dem lebenden Körper (60) hin und in einer Richtung von dem lebenden Körper (60) weg beweglich ist, und
eine Bewegung der Halteteile (12a, 12b, 12c, 12d) eine Auswahl zwischen einem Zustand, in dem die Klebstoffschicht (13) mit dem lebenden Körper (60) in Kontakt ist, und einem Zustand ermöglicht, in dem die Klebstoffschicht (13) von dem lebenden Körper (60) getrennt ist.

4. Montageeinheit (10) nach Anspruch 3, wobei jeder der zwei oder mehr Halteteile (12a, 12b, 12c, 12d) so an dem Hauptkörperteil (11) angebracht ist, dass sie ferner mit Bezug auf den Hauptkörperteil (11) drehbar sind.

5. Montageeinheit (10) nach Anspruch 4, wobei mehrere Klebstoffschichten (13) auf einer Außenfläche von jedem der zwei oder mehr Halteteile (12a, 12b, 12c, 12d) in der Drehrichtung vorgesehen sind.

6. Sensoreinheit (30), die Folgendes umfasst: einen Sensor (20), von dem ein Teil unter der Haut eines lebenden Körpers (60) platziert werden kann; und die Montageeinheit (10) nach Anspruch 1 zum Befestigen des Sensors (20) an dem lebenden Körper (60).

7. Messvorrichtung (50) zum Messen von numerischen Informationen in Bezug auf eine Substanz, die in interstitieller Flüssigkeit und/oder Blut enthalten ist, die sich unter der Haut eines lebenden Körpers (60) befindet/-n, wobei die Vorrichtung (50) Folgendes umfasst:
einen Sensor (20), von dem ein Teil unter der Haut platziert werden kann und der so konfiguriert ist, dass er ein Signal in Abhängigkeit von den numerischen Informationen erzeugt;
die Montageeinheit (10) nach Anspruch 1 zum Befestigen des Sensors (20) an dem lebenden Körper (60); und
eine Steuereinheit (40) zum Empfangen des von dem Sensor (20) erzeugten Signals und zum Ausführen einer Verarbeitung, die Digitalsignalverarbeitung an dem Signal beinhaltet.

8. Messvorrichtung (50) nach Anspruch 7, wobei die Steuereinheit (40) zum Ausführen einer Sendeverarbeitung zum Senden des Signals, das der Digitalsignalverarbeitung unterzogen wurde, zu einer externen Messvorrichtung ausgelegt ist.

9. Sensorbefestigungsverfahren zum Befestigen, an einem lebenden Körper (60), eines Sensors (20), von dem ein Teil unter der Haut des lebenden Körpers (60) platziert werden kann, wobei das Verfahren die folgenden Schritte beinhaltet:
(a) Verwenden einer Montageeinheit (10), die einen Hauptkörperteil (11) und zwei oder mehr an dem Hauptkörperteil (11) angebrachte Halteteile (12a, 12b, 12c, 12d) umfasst, wobei jeder der zwei oder mehr Halteteile (12a, 12b, 12c, 12d) eine Klebstoffschicht (13) aufweist, die an dem lebenden Körper (60) haften kann und am Hauptkörperteil (11) angebracht ist, um eine Auswahl zwischen einem Zustand, in dem die Klebstoffschichten (13) mit dem lebenden Körper (60) in Kontakt sind, und einem Zustand zu ermöglichen, in dem die Klebstoffschichten (13) von dem lebenden Körper (60) getrennt sind, und Halten des Sensors (20) durch den Hauptkörperteil (11) der Montageeinheit (10);
(b) Befestigen der Montageeinheit (10) an dem lebenden Körper (60) durch Inkontaktbringen der Klebstoffschicht (13) von wenigstens einem der zwei oder mehr Halteteile (12a, 12b, 12c, 12d) der Montageeinheit (10) mit dem lebenden Körper (60); und
(c) Bewegen, falls eine der Klebstoffschichten (13) der zwei oder mehr Halteteile (12a, 12b, 12c, 12d) ersetzt werden muss, des Halteteils (12a, 12b, 12c, 12d) einschließlich der zu ersetzenden Klebstoffschicht (13) in einen Zustand, in dem die Klebstoffschicht (13) von wenigstens einem der Halteteile (12a, 12b, 12c, 12d) mit dem lebenden Körper (60) in Kontakt ist, und Lösen der zu ersetzenden Klebstoffschicht (13) von dem lebenden Körper (60).

10. Sensorbefestigungsverfahren nach Anspruch 9,
wobei jeder der zwei oder mehr Halteteile (12a, 12b, 12c, 12d) über einen Gelenkmechanismus (15) an dem Hauptkörperteil (11) angebracht ist,
eine Mobilisierung der Halteteile (12a, 12b, 12c, 12d) mit dem Gelenkmechanismus (15) eine Auswahl zwischen einem Zustand, in dem die Klebstoffschicht (13) mit dem lebenden Körper (60) in Kontakt ist, und einem Zustand ermöglicht, in dem die Klebstoffschicht (13) von dem lebenden Körper (60) getrennt ist, und
in Schritt (c) die zu ersetzende Klebstoffschicht (13) von dem lebenden Körper (60) durch Mobilisieren der Halteteile (12a, 12b, 12c, 12d) mit dem Gelenkmechanismus (15) gelöst wird.

11. Sensorbefestigungsverfahren nach Anspruch 9,
wobei jeder der zwei oder mehr Halteteile (12a, 12b, 12c, 12d) so an dem Hauptkörperteil (11) angebracht ist, dass er in einer Richtung zu dem lebenden Körper (60) hin und in einer Richtung von dem lebenden Körper (60) weg beweglich ist,
eine Bewegung der Halteteile (12a, 12b, 12c, 12d) eine Auswahl zwischen einem Zustand, in dem die Klebstoffschicht (13) mit dem lebenden Körper (60) in Kontakt ist, und einem Zustand ermöglicht, in dem die Klebstoffschicht (13) von dem lebenden Körper (60) getrennt ist, und
in Schritt (c) die zu ersetzende Klebstoffschicht (13) durch eine Bewegung der Halteteile (12a, 12b, 12c, 12d) von dem lebenden Körper (60) gelöst wird.

12. Sensorbefestigungsverfahren nach Anspruch 11, wobei jeder der zwei oder mehr Halteteile (12a, 12b, 12c, 12d) so am Hauptkörperteil (11) angebracht wird, dass er mit Bezug auf den Hauptkörperteil (11) gedreht werden kann.

13. Sensorbefestigungsverfahren nach Anspruch 12,
wobei mehrere der Klebstoffschichten (13) auf einer Außenfläche von jedem der zwei oder mehr Halteteile (12a, 12b, 12c, 12d) in der Drehrichtung vorgesehen sind, und
in Schritt (c) eine neue Klebstoffschicht (13), die die zu ersetzende Klebstoffschicht (13) ersetzt, dem lebenden Körper (60) durch Drehen der Halteteile (12a, 12b, 12c, 12d) zugewandt wird, und danach die neue Klebstoffschicht (13) durch eine Bewegung der Halteteile (12a, 12b, 12c, 12d) auf den lebenden Körper (60) geklebt wird.

## Revendications

1. Unité de montage (10), comprenant :
une portion de corps principal (11) conçue pour maintenir une portion d'un capteur (20) sous la peau d'un corps vivant (60) ; et
deux ou plusieurs portions de maintien (12a, 12b, 12c, 12d) lesquelles sont attachées à la portion de corps principal (11),
cas dans lequel chacune des deux ou plusieurs portions de maintien (12a, 12b, 12c, 12d) comprend une couche de matière adhésive (13) apte à adhérer au corps vivant (60) et est apte à être déplacée de sorte à procurer une sélection entre un état dans lequel la couche de matière adhésive (13) est au contact du corps vivant (60), et un état dans lequel la couche de matière adhésive (13) est séparée du corps vivant (60),
cas dans lequel la portion de corps principal (11) comprend un mécanisme qui peut changer une position de contact d'au moins une des portions de maintien (12a, 12b, 12c, 12d) qui est au contact du corps vivant (60), dans un état dans lequel la position du capteur (20) est maintenue.

2. Unité de montage (10) selon la revendication 1,
chacune des deux ou plusieurs portions de maintien (12a, 12b, 12c, 12d) étant attachée à la portion de corps principal (11) par l'intermédiaire d'un mécanisme à articulation (15), et
la mobilisation des portions de maintien (12a, 12b, 12c, 12d) avec le mécanisme à articulation (15) procurant une sélection entre un état dans lequel la couche de matière adhésive (13) est au contact du corps vivant (60), et un état dans lequel la couche de matière adhésive (13) est séparée du corps vivant (60).

3. Unité de montage (10) selon la revendication 1,
chacune des deux ou plusieurs portions de maintien (12a, 12b, 12c, 12d) étant attachée à la portion de corps principal (11) de sorte à être mobile suivant un sens dirigé vers le corps vivant (60) et suivant un sens s'éloignant du corps vivant (60), et
un mouvement des portions de maintien (12a, 12b, 12c, 12d) procurant une sélection entre un état dans lequel la couche de matière adhésive (13) est au contact du corps vivant (60), et un état dans lequel la couche de matière adhésive (13) est séparée du corps vivant (60).

4. Unité de montage (10) selon la revendication 3, chacune des deux ou plusieurs portions de maintien (12a, 12b, 12c, 12d) étant attachée à la portion de corps principal (11) de sorte à être en outre rotative par rapport à la portion de corps principal (11).

5. Unité de montage (10) selon la revendication 4, une pluralité de couches de matière adhésive (13) étant prévues sur une face externe de chacune des deux ou plusieurs portions de maintien (12a, 12b, 12c, 12d) suivant le sens de rotation.

6. Unité à capteur (30) comprenant : un capteur (20) dont une portion peut être placée sous la peau d'un corps vivant (60) ; et l'unité de montage (10) selon la revendication 1 afin de fixer le capteur (20) sur le corps vivant (60).

7. Appareil de mesure (50) destiné à mesurer des informations numériques relatives à une substance contenue dans au moins l'un des éléments suivants, soit le liquide interstitiel soit le sang, qui se trouve sous la peau d'un corps vivant (60), l'appareil (50) comprenant :
un capteur (20) dont une portion peut être placée sous la peau et qui est configuré de façon à générer un signal en fonction des informations numériques ;
l'unité de montage (10) selon la revendication 1, pour fixer le capteur (20) au corps vivant (60) ; et
une unité de commande (40) conçue pour recevoir le signal ayant été généré par le capteur (20) et pour exécuter un traitement qui inclut un traitement numérique de signaux sur le signal.

8. Appareil de mesure (50) selon la revendication 7, l'unité de commande (40) étant conçue pour exécuter un traitement de transmission afin de transmettre, à un dispositif de mesure externe, le signal qui a été soumis au traitement numérique de signaux.

9. Procédé de fixation d'un capteur pour fixer, sur un corps vivant (60), un capteur (20) dont une portion peut être placée sous la peau d'un corps vivant (60), le procédé comprenant les étapes consistant à :
(a) utiliser une unité de montage (10) laquelle comporte une portion de corps principal (11) et deux ou plusieurs portions de maintien (12a, 12b, 12c, 12d) lesquelles sont attachées à la portion de corps principal (11), chacune des deux ou plusieurs portions de maintien (12a, 12b, 12c, 12d) comprenant une couche de matière adhésive (13) apte à adhérer au corps vivant (60) et est attachée à la portion de corps principal (11) de sorte à procurer une sélection entre un état dans lequel les couches de matière adhésive (13) sont au contact du corps vivant (60), et un état dans lequel les couches de matière adhésive (13) sont séparées du corps vivant (60), et maintenant le capteur (20) par la portion de corps principal (11) de l'unité de montage (10) ;
(b) fixer l'unité de montage (10) au corps vivant (60) en amenant la couche de matière adhésive (13) d'au moins une portion parmi les deux ou plusieurs portions de maintien (12a, 12b, 12c, 12d) de l'unité de montage (10) au contact du corps vivant (60) ; et
(c) dans le cas où l'une quelconque des couches de matière adhésive (13) des deux ou plusieurs portions de maintien (12a, 12b, 12c, 12d) doit être remplacée, déplacer la portion de maintien (12a, 12b, 12c, 12d), incluant la couche de matière adhésive (13) qui a besoin d'être remplacée, dans un état dans lequel la couche de matière adhésive (13) d'au moins une des portions de maintien (12a, 12b, 12c, 12d) est au contact du corps vivant (60), et détacher, du corps vivant (60), la couche de matière adhésive (13) qui a besoin d'être remplacée.

10. Procédé de fixation de capteur, selon la revendication 9,
chacune des deux ou plusieurs portions de maintien (12a, 12b, 12c, 12d) étant attachée à la portion de corps principal (11) par l'intermédiaire d'un mécanisme à articulation (15),
la mobilisation des portions de maintien (12a, 12b, 12c, 12d) avec le mécanisme à articulation (15) procurant une sélection entre un état dans lequel la couche de matière adhésive (13) est au contact du corps vivant (60), et un état dans lequel la couche de matière adhésive (13) est séparée du corps vivant (60), et
lors de l'étape (c), la couche de matière adhésive (13) qui a besoin d'être remplacée étant détachée du corps vivant (60) grâce à la mobilisation des portions de maintien (12a, 12b, 12c, 12d) avec le mécanisme à charnière (15).

11. Procédé de fixation de capteur, selon la revendication 9,
chacune des deux ou plusieurs portions de maintien (12a, 12b, 12c, 12d) étant attachée à la portion de corps principal (11) de sorte à être mobile suivant un sens dirigé vers le corps vivant (60) et suivant un sens s'éloignant du corps vivant (60),
un mouvement des portions de maintien (12a, 12b, 12c, 12d) procurant une sélection entre un état dans lequel la couche de matière adhésive (13) est au contact du corps vivant (60), et un état dans lequel la couche de matière adhésive (13) est séparée du corps vivant (60), et
lors de l'étape (c), la couche de matière adhésive (13) qui a besoin d'être remplacée étant détachée du corps vivant (60) grâce au mouvement des portions de maintien (12a, 12b, 12c, 12d).

12. Procédé de fixation de capteur selon la revendication 11, chacune des deux ou plusieurs portions de maintien (12a, 12b, 12c, 12d) étant attachée à la portion de corps principal (11) de sorte à être rotative par rapport à la portion de corps principal (11).

13. Procédé de fixation de capteur selon la revendication 12,
une pluralité de couches de matière adhésive (13) étant prévues sur une face externe de chacune des deux ou plusieurs portions de maintien (12a, 12b, 12c, 12d) suivant le sens de rotation, et
lors de l'étape (c), une nouvelle couche de matière adhésive (13) remplaçant la couche de matière adhésive (13) qui a besoin d'être remplacée étant dirigée vers le corps vivant (60) grâce à la rotation des portions de maintien (12a, 12b, 12c, 12d), et par la suite la nouvelle couche de matière adhésive (13) étant posée par adhérence sur le corps vivant (60) grâce au mouvement des portions de maintien (12a, 12b, 12c, 12d).
